# EUROPEAN PATENT SPECIFICATION

(11) **EP 2 893 922 B1**
(45) Date of publication and mention of the grant of the patent: **14.07.2021**
(21) Application number: 15150623.5
(22) Date of filing: 09.01.2015
(51) Int. Cl.: A61K 9/50, A61K 9/51, A61K 9/19, A61K 31/12, A61K 31/4965, A61K 31/5575, A61K 33/00, A61P 9/12, A61P 11/00

(54) **Pharmaceutical formulations for the treatment of pulmonary arterial hypertension**
Pharmazeutische Formulierungen zur Behandlung von pulmonaler arterieller Hypertonie
Formulations pharmaceutiques pour le traitement de l'hypertension artérielle pulmonaire

(30) Priority: 10.01.2014 GB 201400412
(43) Date of publication of application: 15.07.2015
(73) Proprietor: Heart Biotech Pharma Limited, Littleton, Winchester SO22 6QA (GB)
(72) Inventor: Yacoub, Magdi, London, WC1N 2EB (GB); El Sherbiny, Ibrahim, 12588 6th of October City (EG)
(74) Representative: Williams, Gareth Owen

(56) References cited:
- WO-A2-2013/123492
- US-A1- 2011 229 580
- US-A1- 2011 250 134
- US-A1- 2013 251 787
- CHI H. LEE: "Treatment of blood flow abnormality using mucosal delivery of nitric oxide", DRUG DELIVERY AND TRANSLATIONAL RESEARCH, vol. 1, no. 3, 30 April 2011 (2011-04-30), pages 201-208, XP055187213, ISSN: 2190-393X, DOI: 10.1007/s13346-011-0026-2
- DU YONG-ZHONG ET AL: "Tumor cells-specific targeting delivery achieved by A54 peptide functionalized polymeric micelles", BIOMATERIALS, vol. 33, no. 34, 6 September 2012 (2012-09-06), pages 8858-8867, XP028942947, ISSN: 0142-9612, DOI: 10.1016/J.BIOMATERIALS.2012.08.043

## Description

### FIELD OF THE INVENTION

The present invention relates to pharmaceutical formulations for the treatment of pulmonary arterial hypertension (PAH). Aspects of the invention relate to inhalable, dry powder formulations for pulmonary administration. Further aspects of the invention relate to formulations suitable for injectable or oral administration.

### BACKGROUND TO THE INVENTION

Pulmonary arterial hypertension (PAH) is a rare but devastating disease, in which the normally low pulmonary artery pressure becomes elevated due to vaso-constriction and to the remodelling of pulmonary vessels. This in turn increases workload on the right side of the heart, causing right heart hypertrophy, fibrosis and ultimately heart failure.

Interventions used in the management of PAH are traditionally targeted on the vasculature, with the aim of enhancing vasodilation and anti-proliferation pathways. These include the prostacyclin analogues and nitric oxide (NO). However, it is increasingly recognized that in addition to the pulmonary vasculature, the right heart is also a viable therapeutic target in the treatment of PAH. PPAR β inhibitors have been shown in recent studies on animal models to reduce right heart hypertrophy without influencing pulmonary vascular remodelling.

### Prostacyclin Analogues

Prostacyclin is a powerful vasodilator, produced in the body by endothelial cells. Patients with PAH are found to have low levels of prostacyclin, leading to a frequently life-threatening constriction of the pulmonary vasculature.

Natural prostacyclin has been found to be unstable in solution, and undergoes rapid degradation, making it very difficult to use for clinical applications. Over 1,000 synthetic prostacyclin analogues have been developed to date as a result. One of these, Treprostinil has demonstrated unique effectiveness in inhibiting platelet activation and as a vasodilator, and it has relatively good stability in solution compared to native prostacyclin. Other prostacyclin analogues, such as Selexipag are currently undergoing clinical trials with highly encouraging results.

Treprostinil is currently marketed in two formulations, as an infusion (subcutaneous or intravenous via a continuous infusion pump), or as an inhaled aerosol, used with a proprietary device, 4 times per day and at least 4 hours apart. The infusion is frequently associated with side effects such as severe site pain or reaction, whilst the aerosol requires a disciplined regimen on the part of patients. Attempts to develop an oral formulation have to date failed FDA regulatory approval due to the relatively adverse risk/efficiency ratio of prototypes tried so far.

Developing alternative formulations of Treprostinil, which bypass the many serious shortcomings of the continuous infusion/pump system, and which allow efficient, controlled, targeted and sustained release of the drug without the complexity of the current aerosol system, should improve patient compliance and experience of treatment, and ultimately impact on successful management of the condition.

Selexipag is currently undergoing Phase III trials. Selexipag is designed to act on IP (prostacyclin) receptors selectively, and has to date demonstrated a significant reduction in pulmonary vascular resistance within the trial cohorts studied. It is currently produced as an oral formulation, and although the administration regime is far simpler than for Treprostinil, again the development of a formulation and delivery mechanism that allows efficient, controlled, targeted and sustained release of the drug would significantly reduce side effects and improve patient compliance, ultimately impacting positively on outcome.

### Nitric Oxide

Nitric oxide is known to have vasodilatory effects on the pulmonary vasculature in both humans and animals. A number of trials suggest that the effects of NO on vascular resistance are selective (ie focused on pulmonary rather than systemic), and do not cause systemic hypotension or raise methaemoglobin.

The main challenge with NO therapy is in sustaining benefit over a prolonged period. A delivery mechanism that allows controlled, sustained release of NO, such as that offered by the present invention, can substantively overcome this challenge. Formulations of NO donors (such as, but not limited to, organic nitrates, nitrite salts, s-nitrosoglutathione (GSNO), and S-Nitrosothiols) can be loaded into the delivery mechanism, which can produce sustained and controlled doses of NO when activated by the moisture in the lung.

### PPAR β Agonists

The pulmonary vasoconstriction and remodelling associated with PAH lead to overloading and hypertrophy of the right heart, and eventually to heart failure. While focusing on vasodilatation is an important part of effective management of PAH, it is increasingly apparent that the hypertrophic right heart is also a valid therapeutic target for intervention in the disease.

The PPARs (peroxisome proliferator activated receptors) have been shown in studies to be an attractive protective pathway in the overloaded heart. As well as protecting against ischemia reperfusion injury, in mice, PPAR has demonstrated effects on reducing left ventricular dilation, fibrosis and mitochondrial abnormalities. GW0742, a ligand which activates PPAR β selectively, has been shown in studies to reduce right heart hypertrophy without influencing pulmonary vascular remodelling. GW0742 is not currently used in the treatment of PAH. Nonetheless it is a feature of this invention that suitable formulations of GW0742 can be developed for loading into nano-particle delivery systems for lung, oral and intravascular administration.

Current treatments for PAH require that the therapy is administered by injection, or by inhalation. Inhalation would typically be preferred over injection, but current inhalable therapies require complex administration forms and schedules, and this has an effect on patient compliance or ease of delivery. Some therapeutics (for example, NO donors) require sustained release for effective administration.

The present invention proposes alternative formulations, which in some embodiments would be easier to administer.

WO 2013/123492 describes Injectable insulin loaded microgels that are capable of modifying the amount of insulin released based on the patient's tissue glucose levels. The microgels contain insulin, glucose oxidase entrapped in or bound to the microgels, and an agent that reduces hydrogen peroxide, entrapped in or bound to the microgels, wherein the polymeric microgel expands when pH decreases from physiological pH and shrinks when pH increases towards physiological pH, thereby releasing insulin at a rate corresponding to the glucose concentration. In one embodiment, the glucose oxidase and/or the agent reducing hydrogen peroxide are encapsulated in nanogels, then encapsulated within the microgel.

US 2011/229580 describes compositions, methods and kits for a microsphere with one or more entrapped nanoparticles. The method of preparation comprises atomizing a suspension comprising a polysaccharide and one or more nanoparticles into a solution comprising a cross linking agent

Du Yong-Zhong et al (2012) "Tumor cell-specific targeting delivery achieved by A54 peptide functionalized polymeric micells", Biomaterials vol 33, no 34, pp 8858-8867 describes a hepatocarcinoma-binding peptide (A54 peptide), incorporated into functionalized and PEGylated stearic acid grafted chitosan (A54-PEG-CS-SA) micelles for targeting therapy of doxorubicin.

US 2013/251787 describes treatment of PAH patients by administering an effective dose of a leukotriene inhibitor. Suitable inhibitors include leukotriene A4 hydrolase (LTA4H) inhibitors, leukotriene B4 receptor (BLT1/BLT2) antagonists, 5-lipoxygenase (5-LO) inhibitors, and 5-lipoxgygenase activating protein (FLAP) inhibitors.

### BRIEF SUMMARY OF THE INVENTION

According to the present invention, there is provided a pharmaceutical formulation for use in the treatment of pulmonary arterial hypertension (PAH), comprising polymeric nanoparticles encapsulated within crosslinked polymeric microparticles, wherein the polymeric nanoparticles carry a therapeutic agent for treatment of PAH loaded within them and selected from the group consisting of prostacyclin synthetic analogs, PPAR β agonists, and NO donors; and wherein the polymeric nanoparticles comprise self-assembly amphiphilic chitosan-PEG-Cholanic acid, or chitosan-PEG-Stearic acid or chitosan-PEG-Oleic acid..

Other features of the invention are described in the dependent claims.

By "nanoparticle" is meant a composition having a mean particle size (preferably diameter) of less than 600 nm, preferably less than 500 nm. Preferably the mean diameter ranges from 1 to 500 nm, more preferably 10-250 nm. In preferred embodiments, the mean diameter is less than 250 nm, preferably less than 200 nm.

By "microparticle" is meant a composition having a mean particle size (preferably diameter) ranging from 0.75 to 10 µm. Preferred mean particle size ranges include 0.75 to 7.5 µm, 1 to 5 µm, 2 to 4 µm. Most preferred range is 1 to 5 µm.

The mean particle size may be determined by any suitable method practiced in the art; examples of suitable methods are exemplified herein.

The nanoparticle plus microparticle formulation described herein may be referred to as a nano-micro-carrier, or nano-micro-particles, or nano-micro formulation.

The present formulations provide therapeutic routes for treatment of PAH which are simpler to administer than existing treatments. This is achieved by incorporating the drug into nanoparticles. However, nanoparticles themselves are difficult to administer by inhalation, so these are then incorporated into crosslinked polymeric microparticles. This makes the formulation suitable for dry powder inhalation. The nanoparticles encapsulate and stabilise the drug, while also allowing for pulmonary delivery due to their size. The microparticles can help to make the physical form more suitable for one or another administration route, while also permitting sustained release of the nanoparticles.

The nanoparticles and microparticles are preferably biodegradable, and are preferably biocompatible. By biodegradable is meant that the particles will break down naturally within the body under physiological conditions; preferably the conditions as found within the lung. By biocompatible is meant that the particles will not elicit an immune response from the patient. The nanoparticles preferably biodegrade under physiological conditions to give a drug release rate of 0.1 - 0.3 w% per hour.

The polymeric nanoparticles comprise a chitosan-derivative polymer. Suitable chitosan-derivative polymers include chitosan-PEG and/or chitosan derivatives having hydrophobic side chains (for example, stearic acid, cholanic acid). Chitosan derivatives are particularly preferred, due to chitosan's ability to enhance absorption in lung tissues through opening the intercellular tight junctions of the lung epithelium. We refer to this ability herein as "epithelial targeting", and this feature also makes similar formulations suitable for administration to other epithelial tissues, for example, the intestine. Thus, in preferred embodiments, the nanoparticles are epithelially targeted.

The polymeric nanoparticles in preferred embodiments have a moisture content, in the dry formulation, of less than 2%.

The polymeric nanoparticles may be produced via emulsion polymerization, ionotropic gelation, polyelectrolyte complexation, and/or self-assembly. In preferred embodiments, the nanoparticles may be produced via self assembly following sonication of amphiphilic polymer solutions.

The cross-linked polymeric microparticles preferably comprise cross-linked hydrogel polymers, and are preferably crosslinked hydrogel microparticles. These may be in the form of semi-interpenetrating polymeric networks (semi-IPNs) and full-IPNs. These semi- and full-IPNs are preferably based on natural polymers such as, but not limited to, chitosan and water soluble chitosan derivatives (such as carboxymethyl and PEGylated derivatives) in a combination with one or more of nontoxic, biocompatible, and biodegradable polymers including, but not limited to, hyaluronate, carrageenan and oligoguluronate. In some embodiments, only chitosan or chitosan derivatives are used. The semi-IPN and IPN microparticles are crosslinked through any suitable method, including ionotropic gelation, polyelectrolyte complexation and/or H-bonding. The nanoparticles-microparticles formulations may be produced using a spray-drying technique, spray gelation, or ionotropic gelation followed by lyophilization. Spray drying is preferred.

The microparticles are preferably swellable; more preferably the microparticles are hydrogel microparticles and the hydrogel is swellable. This allows the hydrogel to absorb moisture from the lung or other delivery site and so permit release of the nanoparticles. The hydrogel is preferably able to swell to at least 200, 300, 400, 500% of the original (dry formulation) size. In a preferred embodiment, a microparticle of 2-5 µm dry diameter is able to swell to at least 20 µm diameter. The microparticle is preferably able to swell to the larger diameter within 10, 9, 8, 7, 6, 5, 4, 3, or 2 minutes from administration to the lungs of a patient. In a further preferred embodiment, the microparticle is able to swell to at least 10 times the dry size within 3 minutes from administration.

The hydrogel preferably comprises less than 10%, preferably less than 7.5%, more preferably less than 5, 4, 3, 2% water when in the dry formulation. In preferred embodiments, this is less than 2%.

The particles preferably biodegrade under physiological conditions (3-5% weight loss per day) to give a drug release rate of less than 1% per hour, more preferably less than 0.5% per hour. In preferred embodiments, the drug release rate is 0.1 - 0.3 w% per hour.

The microparticles may comprise a pH responsive carrier. Smart pH-responsive particles for drug delivery are known, and are used in situations where it is desirable to release a drug from a carrier under certain pH conditions; for example, when the carrier is in a specific environment, such as the intestine. Examples of the preparation and use of pH-responsive carriers are given in PS Stayton and AS Hoffman, "Smart pH-responsive carriers for intracellular delivery of biomolecular drugs", in V Torchilin (ed), Multifunctional Pharmaceutical Nanocarriers, Springer Science and Business Media, 2008; and in Stephanie J. Grainger and Mohamed E. H. El-Sayed, "STIMULI-SENSITIVE PARTICLES FOR DRUG DELIVERY", in Biologically Responsive Hybrid Biomaterials, Esmaiel Jabbari and Ali Khademhosseini (Ed), Artech House, Boston, MA, USA.

The therapeutic agent is selected from prostacyclin synthetic analogs, PPAR β agonists and NO donors. The prostacyclin synthetic analog may be treprostinil or selexipag. The PPAR β agonist may be GW0742. In preferred embodiments, the agent is a prostacyclin synthetic analog, most preferably treprostinil.

The dosage of the therapeutic agent may be selected depending on the desired administration dose to the patient, and may vary depending on the agent to be used, and the composition of the nano and microparticles.

Also disclosed herein is a composition comprising polymeric nanoparticles encapsulated within crosslinked polymeric microparticles, wherein the polymeric nanoparticles carry a therapeutic agent suitable for treatment of PAH loaded within them, for use in the treatment of pulmonary arterial hypertension (PAH). Further disclosed herein is the use of a composition comprising polymeric nanoparticles encapsulated within crosslinked polymeric microparticles, wherein the polymeric nanoparticles carry a therapeutic agent suitable for treatment of PAH loaded within them in the manufacture of a medicament for the treatment of PAH.

Also disclosed herein is a method of treatment of PAH, the method comprising administering a composition comprising polymeric nanoparticles encapsulated within crosslinked polymeric microparticles, wherein the polymeric nanoparticles carry a therapeutic agent suitable for treatment of PAH loaded within them to a patient in need thereof, wherein the composition is administered by inhalation.

Yet further disclosed herein is a pharmaceutical formulation comprising polymeric nanoparticles encapsulated within smart pH-responsive crosslinked polymeric microparticles, wherein the polymeric nanoparticles carry a therapeutic agent selected from prostacyclin synthetic analogs, PPAR β agonists and NO donors loaded within them, wherein the pH-responsive microparticles are targeted to the intestine, and wherein the nanoparticles are targeted to the epithelium. This dformulation may be suitable for intestinal or oral administration, for example. Smart pH-responsive particles for drug delivery are known, and are used in situations where it is desirable to release a drug from a carrier under certain pH conditions; for example, when the carrier is in a specific environment, such as the intestine. Examples of the preparation and use of pH-responsive carriers are given in PS Stayton and AS Hoffman, "Smart pH-responsive carriers for intracellular delivery of biomolecular drugs", in V Torchilin (ed), Multifunctional Pharmaceutical Nanocarriers, Springer Science and Business Media, 2008; and in Stephanie J. Grainger and Mohamed E. H. El-Sayed, "STIMULI-SENSITIVE PARTICLES FOR DRUG DELIVERY", in Biologically Responsive Hybrid Biomaterials, Esmaiel Jabbari and Ali Khademhosseini (Ed), Artech House, Boston, MA, USA.

Further disclosed herein is an injectable pharmaceutical formulation comprising polymeric nanoparticles carrying a therapeutic agent selected from prostacyclin synthetic analogs, PPAR β agonists and NO donors loaded within them, and wherein the nanoparticles are targeted to the epithelium. For injectable formulations, it may not be beneficial to provide microparticles, and the nanoparticles may be injected directly. Although not preferred for treatment of PAH, such formulations may find application where inhalation is not practical for one reason or another, or where sites other than the lungs are to be targeted.

### BRIEF DESCRIPTION OF THE DRAWINGS

Figure 1 shows synthesis of PEG-CS copolymers
Figure 2 shows synthesis of PEG-CS-oleic and PEG-CS-cholanic copolymers
Figure 3 shows characterization and NO release profile from NO nanoparticles
Figure 4 shows the effect of NO nanoparticles on viability and chemokine release from endothelial cells
Figure 5 shows the effect of NO nanoparticles on viability and chemokine release from arterial smooth muscle cells
Figure 6 shows the effect of NO nanoparticles on relaxation of pulmonary arteries in mice
Figure 7 shows the effect of control nanoparticles on viability and chemokine release from endothelial cells
Figure 8 shows the effect of U46619 on pulmonary arteries in mice, and the effect of control nanoparticles on mice
Figure 9 shows the effect of U46619 on pulmonary arteries in mice, and the effect of control nanoparticles and NO nanoparticles on mice.

### DETAILED DESCRIPTION OF THE INVENTION

The present invention provides novel formulations of therapeutics for the treatment of PAH. Therapeutic agents may be selected from prostacyclin synthetic analogs, PPAR β agonists and NO donors. The agents are incorporated into biodegradable polymeric nanoparticles, which themselves are incorporated into hydrogel microparticles or smart pH-responsive microparticles.

Background art which may be of benefit in understanding the invention includes: El-Sherbiny, I.M., & Smyth, H.D.C. (2012). Controlled release pulmonary administration of curcumin using swellable biocompatible nano-microparticles systems. Molecular Pharmaceutics, 9(2), 269-280.

El-Sherbiny IM, and Smyth, HDC. (2010) Biodegradable nano-micro carrier systems for sustained pulmonary drug delivery: (I) self-assembled nanoparticles encapsulated in respirable/swellable semi-IPN microspheres. Int J. Pharm 395: 132-141.
El-Sherbiny IM, Mcgill S, and Smyth HDC. (2010) Swellable microparticles as carriers for sustained pulmonary drug delivery. J. Pharm Sci, 99(5): 2343-2356.
El-Sherbiny IM, and Smyth HDC. (2010) Novel cryomilled physically crosslinked biodegradable hydrogel microparticles as carriers for inhalation therapy. J. Microencapsulation, 27(7): 561-572.
El-Sherbiny IM, Abdel-Mogibb M, Dawidar A, Elsayed A, and Smyth HDC.(2010) Biodegradable pH-responsive alginate-poly (lactic-co-glycolic acid) nano/micro hydrogel matrices for oral delivery of silymarin, CarbohydrPolym, 83, 1345-1354. El-Sherbiny IM, and Smyth HDC. (2010) PLGA nanoparticles encapsulated in respirable/swellable hydrogel microspheres as potential carriers for sustained drug delivery to the lung. Annual Meeting of American Association of Pharmaceutical Scientists, New Orleans, LA.
El-Sherbiny IM, and Smyth HDC. (2010) Nano-micro carrier systems for sustained pulmonary drug delivery. Biomedical Engineering Society Annual Meeting (BMES), Austin, TX. Selvam P, El-Sherbiny IM, and Smyth HDC. (2010) Swellable microparticles for sustained release drug delivery to the lung using propellant driven metered dose inhalers. Biomedical Engineering Society Annual Meeting (BMES), Austin, TX.
El-Sherbiny IM, and Smyth HDC. (2010) Novel non-covalently crosslinked hydrogel nano-microparticles for inhalation therapy.Annual Meeting of American Association of Pharmaceutical Scientists, New Orleans, LA.
El-Sherbiny IM, and Smyth HDC. (2009) Cryomilled physically crosslinked biodegradable hydrogel microparticles as novel potential carriers for inhalation therapy.Annual Meeting of American Association of Pharmaceutical Scientists, Los Angles, CA, AM-09-01692.
El-Sherbiny IM, and Smyth HDC. (2009) Novel spray dried biodegradable semi-IPN hydrogel microspheres for pulmonary drug delivery. Annual Meeting of American Association of Pharmaceutical Scientists, Los Angles, CA, (AM-09-01708).

Reference to these publications should not be taken as an admission that the contents of any particular document are relevant prior art. However, the skilled person is referred to each of these publications for details of ways in which nanoparticles and/or microparticles may be produced.

Various preliminary studies (referred to in the citations listed above) performed with regard to options for inhalation, and oral therapy support the proposal that polymeric nanoparticles and/or nanoparticles-microparticles carrier systems will improve bioavailability, increase targeting, reduce dose frequency, avoid macrophage clearance and confer sustained delivery of therapeutic agents to the lung compared to free drugs. Remarkably, the preliminary data has shown that:
(1) The loading of drugs into polymeric nanoparticles has the potential to significantly enhance dissolution and absorption, and consequently can improve the bioavailability of the loaded drugs.
(2) drug-loaded nanoparticles can be incorporated into novel crosslinked microparticles with physicochemical characteristics (aerodynamic size, shape, moisture content, etc) appropriate for inhalation therapy.
(3) the design and composition of the nanoparticles-microparticles carriers can be modulated to allow them to absorb moisture and expand rapidly to evade endocytosis by macrophage cells.
(4) biodegradation rates of the developed carriers are controllable.
(5) the polymeric nanoparticles-microparticles systems can be used efficiently for dry powder inhalation therapy.
(6) crosslinked polymeric microparticles incorporating drug-loaded nanoparticles can efficiently confer sustained release of these drugs once deposited in the lung.

### HYPOTHESIS

The hypothesis underlying this invention is that the development of appropriate respirable crosslinked polymeric nano-microparticle systems will enhance bioavailability, increase deep lung targeting, reduce dose frequency, avoid macrophage clearance and confer sustained pulmonary delivery of Prostacyclin analogues, Nitric Oxide and PPAR β agonists for the treatment of pulmonary arterial hypertension (PAH) compared to free drugs. Also, that the formulation of the above into pre-designed biodegradable and biocompatible smart pH-responsive hydrogel particles will improve bioavailability, reduce dose frequency, increase targeting, and confer sustained oral and intravascular delivery of these interventions compared to free drugs.

### 1. Composition of Nanoparticles

Our preliminary studies showed that incorporation of drug-loaded nanoparticles into respirable crosslinked microparticles can allow for additional control of drug action and release. Also, in our preliminary investigations, we found that loading of hydrophobic active ingredients into nanoparticles (made of ubiquitous polymers such as PLGA) considerably enhances the dissolution/absorption of these ingredients. Building on these observations, a new series of specifically designed polymeric nanoparticles were obtained via self assembly of a new series of amphiphilic graft and block copolymers. The developed graft and block copolymers are based on natural polymers and chemically modified natural polymers such as, but not limited to, chitosan, chitosan derivatives, alginate, carrageenan, and cellulose derivatives.

Materials: The amphiphilic copolymers were produced via chemical modifications of some natural polymers such as, but not limited to, chitosan and chitosan derivatives through introducing of hydrophilic side chains (such as PEG) and/or hydrophobic moieties (mainly, stearic acid, cholanic acid, phthaloyl, and butyl acrylate). Chitosan, a cationic biopolymer obtained through alkaline N-deacetylation of chitin, has been selected as a preferred base polymer for the development of the nanoparticles due to its numerous desirable characteristics including biodegradability, non-toxicity, biocompatibility, in addition to its ability to enhance drug absorption in lung tissues through opening the intercellular tight junctions of the lung epithelium (see Parka JH, Kwon S, Lee M, Chung H, Kim JH, Kim YS, Park RW, Kim IS, Seo SB, Kwon IC, and Jeong SY. (2006) Self-assembled nanoparticles based on glycol chitosan bearing hydrophobic moieties as carriers for doxorubicin: in vivo biodistribution and anti-tumor activity. Biomaterials, 27: 119-126).

Preparation Method: The self-assembled polymeric nanoparticles were prepared with (and without) sonication of different concentrations of the modified polymer solutionsat different sonication powers (30-75 W) for different intervals. The effect of relative compositions plus the different preparation parameters onto the physicochemical characteristics (mainly particle size) of the resulting nanoparticles were investigated extensively, to ensure desired properties have been achieved.

Drug loading: The nanoparticles of different architectures loaded with the bioactive moieties (treprostinil, Selexipag, PPAR β agonists or NO donor) were prepared in the same manner used for plain nanoparticles. Then, the effect of relative compositions plus the different preparation parameters onto both drug loading capacity and loading efficiency (%) of the produced nanoparticles were studied in detail to determine optimal loading.

### 2. Nanoparticle Characterization

Polymer Characterization: The synthesized and chemically modified polymers used as pre-cursors for the nanoparticles were characterized using different analytical tools such as elemental analysis, FT-IR, differential scanning calorimetry (DSC), and thermogravimetric analysis (TGA). Also, the crystallography patterns of powdered modified polymers were investigated by X-ray diffraction (XRD).

Physicochemical characterization of the developed nanoparticles: The physicochemical properties of the developed nanoparticles such as particle size, moisture content, and morphology were examined using dynamic light scattering, moisture analyzer, scanning electron, and atomic force microscopy. Also, the biodegradation rates of the nanoparticles were estimated. Both plain and cargo (treprostinil, Selexipag, PPAR β agonists or NO donor)-loaded nanoparticles with optimum physicochemical characteristics, drug loading capacity, drug release patterns were selected for further use in the preparation of a series of novel respirable crosslinked polymeric nano-microparticles carriers.

### 3. Microparticle Composition

Based on our preliminary studies, the developed nanoparticles must be encapsulated or formulated into microparticles for pulmonary drug delivery due to their small aerodynamic size, which normally leads to limited deposition in the airways and extensive exhalation from the lungs following inspiration. Also, nanoparticles-based carrier systems may aggregate in both dry powder and liquid forms which causes rapid clearance by macrophage cells. This aspect of the invention develops a range of novel carrier systems for controlling drug delivery (mainly pulmonary) and combines the benefits of both polymeric nanoparticles and the respirable micron-size crosslinked hydrogel particles.

Materials: The respirable microparticles developed comprise of semi-interpenetrating polymeric networks (semi-IPNs) and full-IPNs. These semi- and full-IPNs are based mostly on natural polymers such as, but not limited to, chitosan and water soluble chitosan derivatives (such as carboxymethyl and PEGylated derivatives) in a combination with one or more of nontoxic, biocompatible, and biodegradable polymers including, but not limited to, hyaluronate, carrageenan and oligoguluronate. The semi-IPN and IPN microparticles are crosslinked through ionotropic gelation, polyelectrolyte complexation and/or H-bonding. These microparticles incorporating drug (treprostinil, Selexipag, PPAR β agonists or NO donor)-loaded nanoparticles were produced using spray-drying technique, spray gelation, and ionotropic gelation followed by lyophilization.

### 4. Nano-Microparticle characterization

The design criteria of the nano-microparticle carriers requires geometric and aerodynamic particle sizes for lung delivery (1-5 µm aerodynamic diameter of dry particles), rapid dynamic swelling (>20 µm within minutes), appropriate morphology, reasonable biodegradation rates, low moisture content, high drug (treprostinil, Selexipag, PPAR β agonists or NO donor) loading efficiency and desirable drug release profiles.

The physicochemical properties of the nano-microparticles carriers, such as particle size, moisture content, and morphology were investigated with the aid of dynamic light scattering (DLS), moisture balance, scanning electron microscopy (SEM), and atomic force microscopy (AFM). Particle density and dynamic swelling were determined using pycnometer and laser diffraction, respectively. The loading capacity, and release kinetics of the incorporated treprostinil, Selexipag, PPAR β agonists or NO donor were determined. Also, the biodegradation rates of the developed formulations were measured. Based on the pre-suggested design criteria, the treprostinil-loaded nano-microparticles carriers with optimum physicochemical characteristics, high treprostinil loading efficiency, and desirable in vitro treprostinil release kinetics will be selected and optimized for further in vitro assessment of their delivery performance. Further selection and optimization of the nano-micro carriers will then determine efficacy in the treatment of PAH in an in vivo (rat) model, before further development towards human trials.

### CHARACTERISTICS AND POTENTIAL USES

Improving the physicochemical characteristics of treprostinil via appropriate formulation and targeting would enhance its overall target organ bioavailability.

Incorporation of treprostinil or other drugs suitable for treatment of PAH in polymeric nano-micro-carriers is advantageous over other carriers because of stability, potential for improved permeability across the physiological barriers, increased bioavailability and also reduction of undesirable side effects. In addition, the polymeric nano-micro-carriers can be designed with desirable physicochemical characteristics via the selection of appropriate candidates from a wide range of the available natural and synthetic polymers.

### Examples

### (I) Preparation of plain and therapeutic agent-loaded poly (lactic-co-glycolic acid) nanoparticles:

The plain and therapeutic agents-loaded poly (lactic-co-glycolic acid) [PLGA] nanoparticles were prepared using "single Emulsion/solvent evaporation technique" through a procedure similar to that described in our previous work (see El-Sherbiny IM et al, 2010, Carbohydr Polym, 83, 1345-1354). Briefly, 1g of PLGA was dissolved in 50 ml of methylene dichloride. Then, to this PLGA solution, 5 ml of the therapeutic agent (treprostinil, selexipag, PPAR β agonists or NO donor) solution was added with stirring. A 2.5% w/v aqueous polyvinyl alcohol (PVA) solution (70 ml) was prepared to which, the PLGA/therapeutic agent mixture was added dropwise while vortexing the capping agent (PVA) solution at high setting. The mixture was then sonicated for 2 min at 50% amplitude to create an oil-in-water emulsion. The sonication process was repeated three times until the desired size of the nanoparticles was obtained. The sonication process was performed in an ice-water bath with using pulse function (10 s pulse on, and 10 s pulse off) in order to evade the heat built-up of the PLGA/therapeutic agent solution during the sonication. Afterwards, the emulsion was immediately poured into 100 ml of an aqueous 0.3% w/v PVA solution with rapid stirring. The resulting PLGA nano-emulsion was stirred overnight in uncovered container to allow for methylene chloride and ethanol evaporation. The resulting PLGA NPs aqueous suspension was used directly or further used in the preparation of the nano-in-microparticles for inhalation. The prepared PLGA nanoparticles showed dense, compact, and integrated spherical shapes with particle radius of 247 ± 10 and 271 ± 18 nm for the plain and the drug-loaded PLGA nanoparticles, respectively, as determined by DLS.

### (II) Preparation of plain and therapeutic agent-loaded chitosan-based nanoparticles:

### (1) Preparation of PEG-grafted-CS copolymer

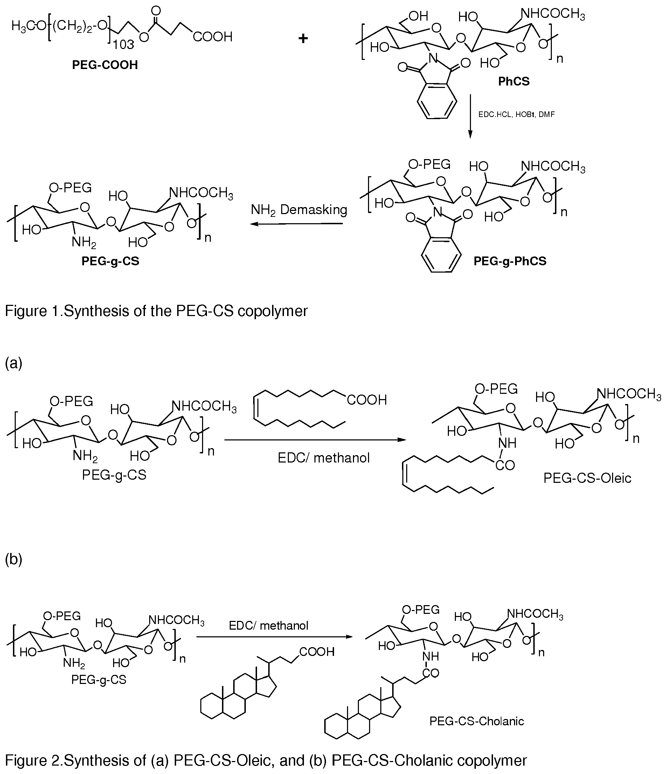

The copolymer of PEG grafted onto CS was prepared (as illustrated in Figure 1) by a modified method of that reported in our earlier study (El-Sherbiny, I.M., & Smyth, H.D.C. (2012). Controlled release pulmonary administration of curcumin using swellable biocompatible nano-microparticles systems. Molecular Pharmaceutics, 9(2), 269-280)and described briefly as follows:
(i) Preparation of PEG-COOH: methoxy-PEG (100 g, 20 mmol), 4-dimethylaminopyridine, DMAP (2.44 g, 20 mmol), triethylamine (2.02 g, 20 mmol), and succinic anhydride (2.4 g, 24 mmol) were dissolved in 300 ml of dry dioxane. The mixture was stirred at room temperature for 2 days under a dry nitrogen atmosphere. Dioxane was then evaporated under vacuum and the residue was taken up in CCl₄, filtered and precipitated by diethyl ether to produce PEG-COOH powder. (ii) Masking of the NH₂ groups of CS: phthalic anhydride (44.8 g, 5 molequivalent to pyranose rings) was reacted with 10 g of CS in 150 ml of DMF at 130°C under inert atmosphere for 10 h. The resulting phthaloyl CS (PhCS) was then collected by filtration after precipitation on ice, washed extensively with methanol, and dried at 45°C under vacuum to produce the yellowish brown PhCS. (iii) Conjugation of PEG-COOH with PhCS: PEG-COOH (37.9 g) was stirred with PhCS (5.0 g, 0.4 mol equivalent to PEG-COOH) in 70 ml of DMF. Then, 1-hydroxybenzotrizole, HOBt (3.4 g, 3 mol equivalent to PEG-COOH) was added with stirring at room temperature until a clear solution was obtained. The 1-ethyl-3-(3-dimethylaminopropyl) carbodiimide hydrochloride, EDC.HCI (4.25 g, 3 mol equivalent to PEG-COOH) was then added with stirring the mixture overnight at room temperature. A purified PEG-g-PhCS copolymer (5.47g, white product) was obtained after dialysis of reaction mixture against distilled water followed by washing with ethanol. (iv) Demasking of PEG-g-PhCS: PEG-g-PhCS (4.1 g) was heated up to 100°C with stirring under inert atmosphere in 20 ml of DMF. Then, 15 ml of hydrazine hydrate was added and the reaction was continued for 1.5 h. The resulting PEG-g-Cs was purified by dialysis against a (1:1) mixture of ethanol and deionized water then dried under vacuum at 45°C.

### 2. Preparation of PEG-CS-Oleic and PEG-CS-Cholanic acid copolymers:

Hydrophobic moieties including oleic, and cholanic acid were coupled to CS backbone of the PEG-g-CS by formation of amide linkages through the EDC-mediated reaction as follows (Figure 2): Briefly, PEG-g-CS (1 g) was dissolved in 0.6% (w/v) aqueous acetic acid solution (100 ml) and diluted with 85 ml methanol. HM was then added to PEG-g-CS solution at 0.4-0.5 mol/l glucosamine residue of CS followed by a drop-wise addition of 15 ml EDC methanol solution (0.07 g/l) while stirring at room temperature. After 20 h, the reaction mixture was added to 200 ml of methanol/ammonia solution (7/3, v/v) while stirring. The precipitated material was filtered; washed with distilled water, methanol, and ether; and then dried under vacuum for 20 h at room temperature. The DS, which represents the number of HM groups per 100 amino groups of CS, was evaluated using normal titration.

### 3. Characterization of the modified polymers:

The synthesized and chemically modified polymers used as pre-cursors for the nanoparticles fabrication were characterized using several analytical techniques such as elemental analysis (EA), Fourier transform infrared (FT-IR), nuclear magnetic resonance (NMR), differential scanning calorimetry (DSC), and thermogravimetric analysis (TGA). Also, the crystallography patterns of powdered modified polymers were investigated by X-ray diffraction (XRD).

### 4. FT-IR and elemental analysis data of some of the developed polymers and copolymers

*PhCS:* FT-IR (vₘₐₓ, cm⁻¹) 3286, 2972, 1770, 1689, 1401, 1050, 727; (C₈H₁₃NO₅)_{0.2363}(C₆H₁₁NO₄)_{0.016}(C₁₄H₁₃NO₆)_{0.747}, calculated (%) *(DS* = 0.97) (%): C 55.71, H 4.86, N 5.21; found (%), C 60.27, H 4.80, N 4.97. *PEG-COOH:* FT-IR (vₘₐₓ, cm⁻¹) 3502, 2879, 1743, 1114; (C₂₃₁H₄₆₁O₁₁₇), calculated (%): C 54.38, H 9.04; found (%), C 56.3, H 9.21. *PEG-PhCS copolymer:* FT-IR (vₘₐₓ, cm⁻¹) 3411, 2901, 1739, 1712, 1091, 720, found EA (%), C 56.21, H 4.61, N 5.22. *PEG-CS copolymer:* FT-IR (vₘₐₓ, cm⁻¹) 3305, 2871, 1706, 1099; found EA (%), C 40.51, H 4.74, N 14.09.

### 5. Development of plain and drug-loaded modified CS-based self-assembled nanoparticles:

The developed modified CS copolymers (PEG-g-CS, PEG-CS-Oleic, and PEG-CS-Cholanic) were used to develop a new series of self-assembled nano-carrier systems for the controlled sustained delivery of treprostinil, selexipag, PPAR β agonists and NO. The nanoparticles were prepared with and without sonication of different concentrations (0.05-1.5%) of the modified polymers solutions using probe sonicator. The sonication process was performed at different sonication powers (20-45 W) for different intervals (30-120 s). The effect of relative compositions plus the different preparation parameters onto the physicochemical characteristics (particle size, morphology, drug loading capacity, and moisture content) of the resulting nanoparticles was investigated. The prepared self-assembled nanoparticles showed particle radius of 95 ± 7 and 105 ± 13 nm for the plain and the drug-loaded nanoparticles, respectively, as determined by DLS.

### 6. Development of plain and drug-loaded modified CS-based hydrogel nanoparticles:

The prepared CS copolymers (PEG-g-CS, PEG-CS-Oleic, and PEG-CS-Cholanic) were used to develop new series of hydrogel nano-carrier systems for the controlled sustained delivery of treprostinil, selexipag, PPAR β agonists and NO. This has been achieved using various types of crosslinkers (mainly tripolyphosphate, TPP and genipin). The preparation was carried out in a mild aqueous media to ensure the stability of the loaded therapeutic agents (treprostinil, selexipag, PPAR β agonists and NO-donor). The prepared self-assembled nanoparticles showed particle radius of 295 ± 19 and 311 ± 24 nm for the plain and the drug-loaded nanoparticles, respectively, as determined by DLS.

### 7. Development of plain and drug-loaded respirable nano-microparticles:

The developed PLGA, self-assembled or hydrogel nanoparticles loaded with treprostinil, selexipag, PPAR β agonists or NO-donor were incorporated into respirable semi-interpenetrating polymeric networks (semi-IPNs) or full-IPNs microparticles. These semi- and full-IPNs were based mostly onto CS derivatives (such as N-trimethyl CS, carboxymethyl CS, and PEGylated CS) in a combination with one or more of nontoxic, biocompatible, and biodegradable polymers including, sodium alginate, hyaluronate, carrageenan and oligoguluronate. The semi-IPN and IPN microparticles were crosslinked through ionotropic gelation, polyelectrolyte complexation and/or H-bonding. The microparticles incorporating drug (treprostinil, Selexipag, PPAR β agonists or NO donor)-loaded nanoparticles were produced using spray gelation, ionotropic gelation followed by lyophilization, or spray-drying technique (NANO-01A nano-spray dryer, MECC CO., LTD., Japan). The developed nano-microparticles powder showed desirable swelling extents (10-24 times of dry size) within three minutes and also showed accepted moisture contents as dry powders (less than 1.6%), good enzymatic degradation rates, promising drug loading capacity (more than 90%), and consistent sustained release of the loaded therapeutic agents.

### Experiments

Nanoparticles were prepared as described above from a combination of polymers including chitosan of low molecular weight, PEG 400 and polyvinyl pyrolidone (PVP), and other materials such as glucose and tetramethyl orthosilicate. Particles were either loaded with an NO donor (referred to as "NO nanoparticles") or not ("control" or "plain" nanoparticles). Glucose was used to perform a thermal reduction of the loaded NO-donor, sodium nitrite (NaNO₂) to generate NO gas. The NO remain entrapped inside the nanoparticles powder until undergoing a sustained release because of the swelling of the prepared hydrogel-based nanoparticles upon exposure to moist environment. Various nanoparticles were prepared with different concentrations of the NO-donor, sodium nitrite. In the following experiments, a sample referred to as "NO-Polymer 4" was used. This was prepared as follows: Sodium nitrite solution was prepared (1g in 30 mM PBS at pH 7.5). Then, D-glucose was added at 40 mg D-glucose/mL of sodium nitrite solution followed by addition of PVP (6.25 mg) with stirring. Afterwards, PEG-400 was added at 0.5 mL PEG/10 mL of solution. Acidic chitosan solution (0.5% w/v) of low molecular weight was then added at a ratio of 0.5 mL/10 mL of sodium nitrite solution. In another container, acidic solution of tetramethyl orthosilicate (2.5 mL/ 0.6 ml HCI) was prepared and sonicated in ice bath, then added to the NO-donor solution (1 mL tetramethyl orthosilicate /10mL solution). The resulting mixture was then stirred and set aside for gelation, followed by freeze drying. The control nanoparticles were prepared using the same procedures but without the NO-donor.

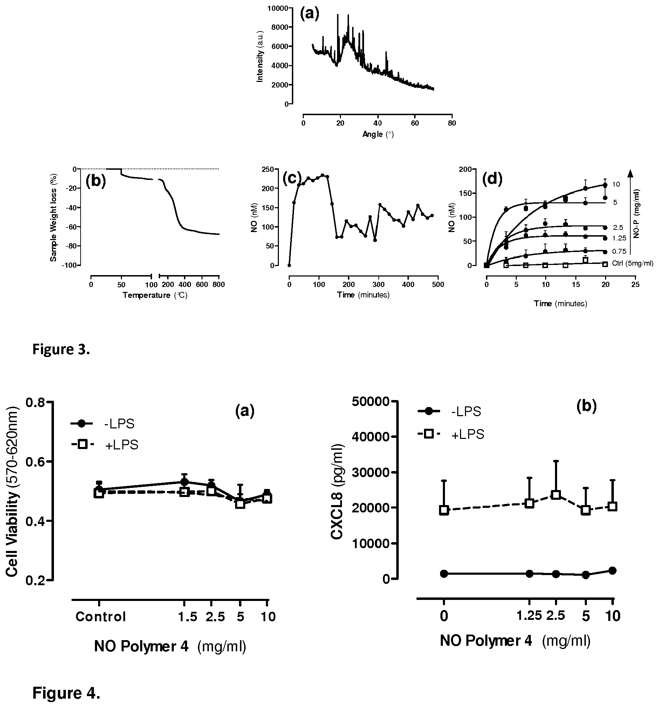

The nanoparticles were characterised by powder X-ray diffraction (Figure 3a) and themogravimetric analysis (Figure 3b). Particles were also assessed for NO release over a period of 8 hours (Figure 3c) and NO release over 20 minutes from different concentrations of NO nanoparticles (0.75, 1.25, 2.5, 5, and 10 mg/ml) compared to control plain nanoparticles (at 5 mg/ml). It can be seen that the majority of NO is released in the first 100 or so minutes, while higher concentrations of nanoparticles release more NO.

Figure 4 shows the effect of the NO nanoparticles used in Figure 3 on viability (Figure 4a) and release of the chemokine CXCL8 (Figure 4b) from endothelial cells grown from blood of healthy donors with or without stimulation with LPS. Nanoparticles were contacted with the cells at concentrations of 1.5, 2.5, 5, and 10 mg/ml.

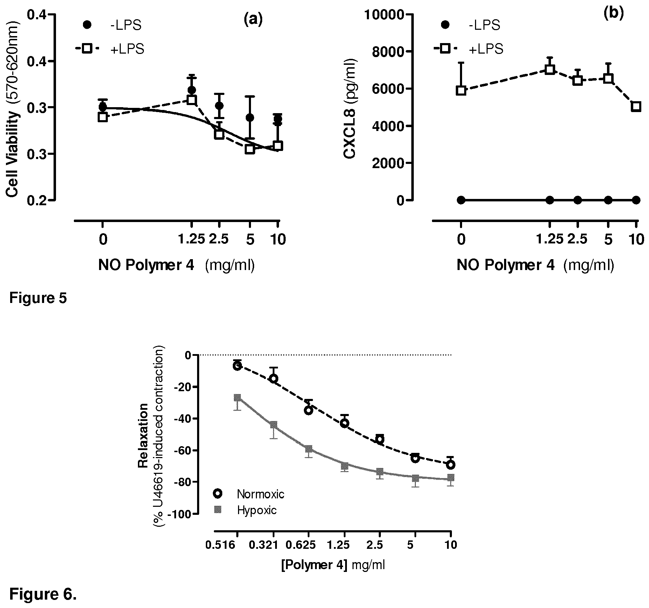

Figure 5 shows a similar experiment to Figure 4, using pulmonary artery smooth muscle cells (PAVSMCs) in place of endothelial cells.

Figure 6 shows the effect of the NO nanoparticles at different concentrations on the relaxation of pulmonary arteries from control mice (normoxic) and mice with pulmonary hypertension (hypoxic). Relaxation is shown as a percentage of U46619-induced contraction. It can be seen that the NO nanoparticles induce substantial relaxation in both mouse groups.

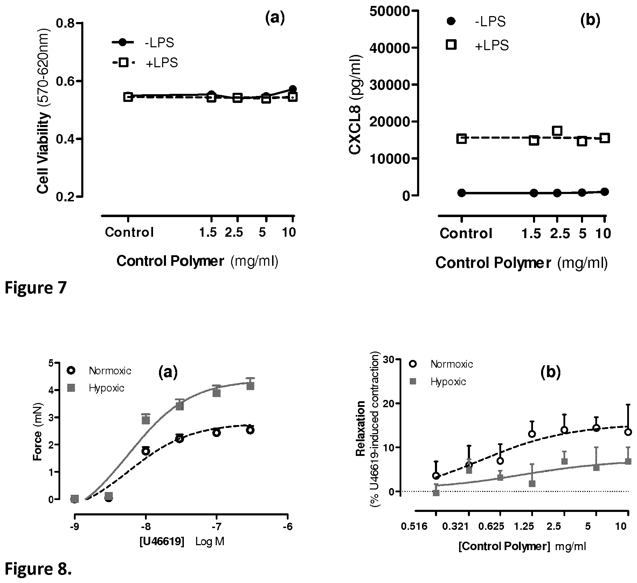

Figure 7 shows the effect of control nanoparticles on viability (Figure 7a) and release of the chemokine CXCL8 (Figure 7b) from endothelial cells grown from blood of healthy donors with or without stimulation with LPS. Nanoparticles were contacted with the cells at concentrations of 1.5, 2.5, 5, and 10 mg/ml. There is essentially no effect of the nanoparticles lacking NO.

Figure 8 shows the effect of U46619 on pulmonary arteries from control mice (Normoxic) and those with pulmonary hypertension (Hypoxic) (Figure 8a), and the effect of control nanoparticles (NO-free polymer nanoparticles) on relaxing pulmonary arteries from hypoxic vs normoxic mice that have been pre-contracted with U46619 (Figure 8b). U46619 (9,11-Dideoxy-9α,11α-methanoepoxy prostaglandin F2a) is a stable synthetic analog of the endoperoxide prostaglandin PGH2, and is a vasoconstrictor that mimics the hydroosmotic effect of vasopressin.

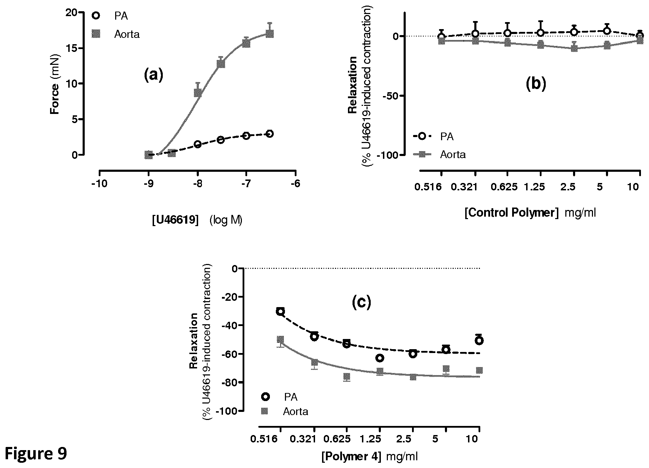

Figure 9 shows the effect of U46619 on contractile responses of pulmonary arteries and aorta from control mice (normoxic) (Figure 9a). There is a significant contraction of the aorta, with less contraction of the pulmonary artery. Figures 9b and 9c show respectively the effect of control nanoparticles (no NO) and NO nanoparticles on relaxing pulmonary arteries and aorta from control mice that have been pre-contracted with U46619. There is a substantial relaxation effect from the NO nanoparticles, which is not seen with the control nanoparticles.

Thus, these experiments demonstrate that NO-releasing agents can be incorporated into nanoparticles, and that these nanoparticles will induce relaxation of pulmonary arteries and aorta in a mouse model.

## Claims

1. A pharmaceutical formulation for use in the treatment of pulmonary arterial hypertension (PAH), comprising polymeric nanoparticles encapsulated within crosslinked polymeric microparticles, wherein the polymeric nanoparticles carry a therapeutic agent for treatment of PAH loaded within them and selected from the group consisting of prostacyclin synthetic analogs, PPAR β agonists, and NO donors; and wherein the polymeric nanoparticles comprise self-assembly amphiphilic chitosan-PEG-Cholanic acid, or chitosan-PEG-Stearic acid or chitosan-PEG-Oleic acid.

2. The formulation for use of claim 1 which is a) an inhalable, dry powder pharmaceutical formulation; or b) an oral pharmaceutical formulation; or c) an injectable pharmaceutical formulation.

3. The formulation for use of any preceding claim wherein a) the nanoparticles have a mean particle diameter of from 1 to 500 nm; and/or b) wherein the crosslinked microparticles have a mean particle diameter from 1 to 5 µm for inhalable formulations, and from 1 to 500 µm, most preferably from 1 to 250 µm for oral formulations.

4. The formulation for use of any preceding claim wherein the microparticles comprise a pH responsive carrier.

5. The formulation for use of any preceding claim wherein a) the polymeric nanoparticles are produced via self assembly following sonication of amphiphilic polymer solutions; and/or b) wherein the microparticles incorporating nanoparticles are produced using spray-drying technique, spray gelation, or ionotropic gelation followed by lyophilization.

6. The formulation for use of any preceding claim wherein the crosslinked polymeric microparticles are cross-linked hydrogel polymeric microparticles; preferably wherein the polymeric hydrogel microparticles are pH-responsive and comprise semi-interpenetrating polymeric networks (semi-IPNs) or full-IPNs.

7. The formulation for use of any preceding claim wherein the microparticles comprise chitosan or a water soluble chitosan derivative, such as carboxymethyl and PEGylated derivatives; preferably wherein the microparticles further comprise one or more polymers selected from hyaluronate, carrageenan and oligoguluronate.

8. The formulation for use of any preceding claim wherein the microparticles are able to swell to at least 500% of the original (dry formulation) size, and/or wherein the formulation is an inhalable dry powder formulation, and the microparticle is able to swell to a larger diameter within 10 minutes from administration to the lungs of a patient.

9. The formulation for use of any preceding claim wherein the therapeutic agent is a prostacyclin synthetic analog, most preferably treprostinil or selexipag.

## Patentansprüche

1. Pharmazeutische Formulierung zur Verwendung in der Behandlung von pulmonaler arterieller Hypertonie (PAH), umfassend in vernetzten polymeren Mikropartikeln verkapselte polymere Nanopartikel, wobei die polymeren Nanopartikel ein therapeutisches Mittel zur Behandlung von PAH tragen, das in ihnen geladen ist und ausgewählt ist aus der Gruppe bestehend aus Prostacyclin synthetischen Analoga, PPARbeta Agonisten und NO-Donoren; und wobei die polymeren Nanopartikel selbstassemblierende amphiphile Chitosan-PEG-Cholansäure oder Chitosan-PEG-Stearinsäure oder Chitosan-PEG-Ölsäure umfassen.

2. Formulierung zur Verwendung nach Anspruch 1, die a) eine inhalierbare, trockene, pulverförmige pharmazeutische Formulierung ist; oder b) eine orale pharmazeutische Formulierung ist; oder c) eine injizierbare pharmazeutische Formulierung ist.

3. Formulierung zur Verwendung nach einem der vorhergehenden Ansprüche, wobei a) die Nanopartikel einen mittleren Partikeldurchmesser von 1 bis 500 nm aufweisen, und/oder b) wobei die vernetzten Mikropartikel für inhalierbare Formulierungen einen mittleren Partikeldurchmesser von 1 bis 5 µm, und für orale Formulierungen von 1 bis 500 µm, am meisten bevorzugt von 1 bis 250 µm, aufweisen.

4. Formulierung zur Verwendung nach einem der vorhergehenden Ansprüche, wobei die Mikropartikel einen auf pH-Wert ansprechenden Träger umfassen.

5. Formulierung zur Verwendung nach einem der vorhergehenden Ansprüche, wobei a) die polymeren Nanopartikel via Selbst-Assemblierung nach Beschallung amphiphiler Polymerlösungen erzeugt werden; und/oder b) wobei die Nanopartikel inkorporierenden Mikropartikel unter Anwendung von Sprühtrocknungstechnik, Sprühgelierung oder ionotroper Gelbildung und anschließender Lyophilisierung hergestellt werden.

6. Formulierung zur Verwendung nach einem der vorhergehenden Ansprüche, wobei die vernetzten polymeren Mikropartikel vernetze polymere Hydrogel-Mikropartikel sind; bevorzugt wobei die polymeren Hydrogel-Mikropartikel auf pH-Wert ansprechen und halb interpenetrierende polymere Netzwerke (Semi-IPNs) oder Voll-IPNs umfassen.

7. Formulierung zur Verwendung nach einem der vorhergehenden Ansprüche, wobei die Mikropartikel Chitosan oder ein wasserlösliches Chitosan-Derivat umfassen, wie beispielsweise Carboxymethyl- und PEG-ylierte Derivate; bevorzugt wobei die Mikropartikel ferner ein oder mehrere Polymere umfassen, die ausgewählt sind aus Hyaluronat, Carrageenan und Oligoguluronat.

8. Formulierung zur Verwendung nach einem der vorhergehenden Ansprüche, wobei die Mikropartikel in der Lage sind, um mindestens 500% der ursprünglichen (Trockenformulierung) Größe zu quellen, und/oder wobei die Formulierung eine inhalierbare Trockenpulver-Formulierung ist und das Mikropartikel in der Lage ist, innerhalb von 10 Minuten nach Verabreichung an die Lunge eines Patienten auf einen größeren Durchmesser zu quellen.

9. Formulierung zur Verwendung nach einem der vorhergehenden Ansprüche, wobei das therapeutische Mittel ein synthetisches Prostacyclin-Analogon ist, am meisten bevorzugt Treprostinil oder Selexipag.

## Revendications

1. Formulation pharmaceutique pour une utilisation dans le traitement d'une hypertension artérielle pulmonaire (PAH), comprenant des nanoparticules polymères encapsulées dans des microparticules polymères réticulées, dans laquelle les nanoparticules polymères portent un agent thérapeutique pour le traitement d'une PAH chargé dans celles-ci et choisi dans le groupe constitué de : analogues synthétiques de la prostacycline, agonistes de PPAR β et donneurs de NO ; et dans laquelle les nanoparticules polymères comprennent un auto-assemblage de chitosane-PEG-acide cholanique ou chitosane-PEG-acide stéarique ou chitosane-PEG-acide oléique amphiphiles.

2. Formulation pour une utilisation selon la revendication 1, qui est a) une formulation pharmaceutique sous forme de poudre sèche, inhalable ; ou b) une formulation pharmaceutique orale ; ou c) une formulation pharmaceutique injectable.

3. Formulation pour une utilisation selon l'une quelconque des revendications précédentes, dans laquelle a) les nanoparticules ont un diamètre de particules moyen de 1 à 500 nm ; et/ou b) dans laquelle les microparticules réticulées ont un diamètre de particules moyen de 1 à 5 µm pour des formulations inhalables, et de 1 à 500 µm, le plus préférablement de 1 à 250 µm, pour des formulations orales.

4. Formulation pour une utilisation selon l'une quelconque des revendications précédentes, dans laquelle les microparticules comprennent un support sensible au pH.

5. Formulation pour une utilisation selon l'une quelconque des revendications précédentes, dans laquelle a) les nanoparticules polymères sont produites via un auto-assemblage à la suite d'une sonication de solutions de polymères amphiphiles ; et/ou b) dans laquelle les microparticules incorporant des nanoparticules sont produites en utilisant une technique de séchage par atomisation, une gélification par atomisation ou une gélification ionotropique suivie d'une lyophilisation.

6. Formulation pour une utilisation selon l'une quelconque des revendications précédentes, dans laquelle les microparticules polymères réticulées sont des microparticules polymères d'hydrogel réticulées ; de préférence dans laquelle les microparticules d'hydrogel polymères sont sensibles au pH et comprennent des réseaux polymères semi-interpénétrants (semi-IPNs) ou totalement-IPNs.

7. Formulation pour une utilisation selon l'une quelconque des revendications précédentes, dans laquelle les microparticules comprennent du chitosane ou un dérivé de chitosane soluble dans l'eau, tels que des dérivés de carboxyméthyle et PEGylés ; de préférence dans laquelle les microparticules comprennent en outre un ou plusieurs polymères choisis parmi : hyaluronate, carraghénane et oligoguluronate.

8. Formulation pour une utilisation selon l'une quelconque des revendications précédentes, dans laquelle les microparticules sont capables de gonfler pour atteindre au moins 500% de la taille (formulation sèche) d'origine et/ou dans laquelle la formulation est une formulation sous forme de poudre sèche, inhalable et la microparticule est capable de gonfler pour atteindre un diamètre plus élevé dans les 10 minutes à partir de l'administration aux poumons d'un patient.

9. Formulation pour une utilisation selon l'une quelconque des revendications précédentes, dans laquelle l'agent thérapeutique est un analogue synthétique de la prostacycline, le plus préférablement tréprostinil ou sélexipag.
